# EUROPEAN PATENT APPLICATION

(11) **EP 4 722 198 A1**
(43) Date of publication of application: **08.04.2026**
(21) Application number: 24204772.8
(22) Date of filing: 04.10.2024
(51) Int. Cl.: C07D 239/95, C07D 407/12, A61P 31/04, A61K 31/517, C07D 491/056

(54) **COMPOUNDS FOR USE IN THE TREATMENT OF BACTERIAL DISEASES AND INFECTIONS**

(71) Applicant: Technische Universität München, in Vertretung des Freistaats Bayern, 80333 München (DE)
(72) Inventor: SIEBER, Stephan, 86191 Utting (DE); GLEIßNER, Carolin, 81479 München (DE); MACSICS, Robert, 85748 Garching (DE)
(74) Representative: Engelhard, Markus

(57) **Abstract**

The present invention relates to compounds having a structure according to formula I and to pharmaceutical compositions comprising at least one of said compounds. The present invention further relates to the use of the compounds in the treatment of a bacterial disease or bacterial infections or as disinfectant. The present invention further relates to a method of treating a bacterial disease or bacterial infections.

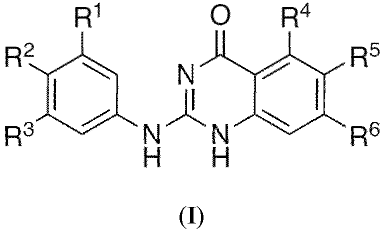

## Description

The present invention relates to compounds having a structure according to formula I and to pharmaceutical compositions comprising at least one of said compounds. The present invention further relates to the use of the compounds in the treatment of a bacterial disease or bacterial infections or as disinfectant. The present invention further relates to a method of treating a bacterial disease or bacterial infections.

### BACKGROUND OF THE INVENTION

The spread of antibacterial resistance has been deemed one of the greatest challenges of mankind by the world's leaders at the 2017 G20 summit (G20 Leaders Declaration Shaping an interconnected world. (2017)). Latest numbers published by the European Center for Disease Prevention and Control (ECDC) showed that the number of deaths caused by infections with antibiotic-resistant bacteria is still rising throughout Europe (Cassini *et al*., 2018).

The rise of multi-drug resistant bacterial pathogens poses a severe threat to human health. In particular, multi-resistant strains of the opportunistic pathogen *Staphylococcus aureus* have become a global problem. In the US 14 % (11,285 out of 80,461) of patients hospitalized with invasive methicillin-resistant *S. aureus* (MRSA) infections died in 2011 (Antibiotic Resistance Threats in US, 2013). In the same time 20,000 cases of Vancomycin-resistant Enterococci infections occurred in the US with 1,300 attributable deaths. Such infections are often left with no treatment options available.

Given this scenario, it is surprising to note that with the exception of linezolid and the lipopeptide daptomycin there have been no novel antibacterial classes of clinical relevance discovered since 1970 (Lakemeyer *et al*., 2018). This innovation gap combined with the emergence of bacterial strains resistant to current antibiotics is the main reason for the current crisis of antibacterial chemotherapy that threatens to return the treatment of bacterial infections to a pre-antibiotic era. In the face of this crisis many resources have been committed to improve existing antibiotic classes. However, it was recently suggested to refocus on the identification of new classes of antibiotic lead structures that address novel yet unexploited cellular targets, rather than further optimize existing antibiotics that address a limited set of bacterial targets (bacterial cell wall biosynthesis, protein biosynthetic pathways, folate coenzyme biosynthesis, and DNA replication and repair).

The rapid emergence of resistance of pathogens has many reasons including the widespread use of antibiotics due to over-prescription and inappropriate, extensive use in the farming industry. Bacteria have developed several strategies to evade antibiotics: destruction of the antibiotic by bacterial enzymes, target modification and the reduction of the antibiotic concentration within the cell by either an increased expression of efflux pumps, restricted penetration and/or by overexpression of the cellular target (Lakemeyer *et al*., 2018). An effect called cross-resistance leads to the development of resistance to a given antibiotic even without direct exposure of the bacteria to it. This is due to the limited set of currently exploited bacterial targets, which might mutate under treatment with a different antibiotic and therefore become insensitive to several antibiotics at the same time.

As described in published International Patent Application No. WO 2017/207556 A2, novel N-N'- bis-aryl ureas have been identified as highly potent antibacterial agents with beneficial properties such as anti-biofilm, anti-persister activity and lack of resistance development. Furthermore, first pharmacokinetic studies and efficacy studies in mice showed the great potential of the lead structure PK150 (also referred to as aBA024) for development into a systemic antibiotic with great market potential. Bis-aryl ureas such as Triclocarban have been successfully marketed as topical antibacterials and additives in cosmetics for many years (Beaver *et al*., 1957; European Commission. Opinion on Triclocarban, 2005). Furthermore several works have described N-N'- bis-aryl ureas and derivatives thereof as antibacterial agents over the past years (Yoon *et al*., 2002; Seth et al., 2004; Pujol *et al.,* 2018; Proctor *et al*., 2002; Chang *et al*., 2016). However, no such compound has yet been developed into a systemic antibiotic.

Thus, there is a great need for new small compounds having antibacterial activity for the treatment of bacterial diseases, in particular for the treatment of bacterial diseases caused by bacteria resistant to commonly used antibiotic agents.

It is an objective of the present invention to provide novel antibacterial compounds and their use as a medicament.

### SUMMARY OF THE INVENTION

According to the present invention this object is solved by providing a compound having a structure according to formula I 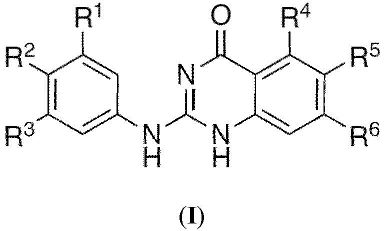 wherein
**R¹**, **R²** and **R³** are each independently selected from H, halogen, cyano, substituted or unsubstituted (C₁-C₆)alkyl, and (C₁-C₆)haloalkyl, under the proviso that not all of **R¹** to **R³** are each H, or
**R²** and **R³** are taken together to form a substituted or unsubstituted cyclic acetal,
**R⁴**, **R⁵** and **R⁶** are each independently selected from H, -O**R⁷**, (C₁-C₆)alkoxy, halogen, substituted or unsubstituted (C₁-C₆)alkyl, (C₁-C₆)haloalkyl, cycloalkyl, substituted or unsubstituted (C₆-C₁₄)aryl(C₁-C₆)alkyl, and - C(O)-**X-R⁸** under the proviso that not all of **R⁴** to **R⁶** are each H, or
**R⁵ and R⁶** are taken together to form a substituted or unsubstituted cyclic acetal;
**R⁷** is selected from H, (C₁-C₆)alkyl, (C₁-C₆)haloalkyl, and substituted or unsubstituted (C₆-C₁₄)aryl,
X is O or NH,
**R⁸** is selected from substituted or unsubstituted (C₁-C₆)alkyl, cycloalkyl, (C₁-C₆)haloalkyl and substituted or unsubstituted (C₆-C₁₄)aryl;
or a pharmaceutically acceptable salt, solvate or hydrate thereof.

According to the present invention this object is solved by providing a pharmaceutical composition comprising
(i) at least one compound according to the present invention,
(ii) optionally, pharmaceutically excipient(s) and/or carrier.

According to the present invention this object is solved by providing the compound of the present invention or the pharmaceutical composition of the present invention for use as a medicament.

According to the present invention this object is solved by providing the compound of the present invention or the pharmaceutical composition of the present invention for use in the treatment of a bacterial disease or bacterial infection.

According to the present invention this object is solved by a disinfectant comprising at least one compound of the present invention.

According to the present invention this object is solved by a method for the treatment of a bacterial disease or bacterial infections, comprising the step of administering to a subject in need thereof a compound of the present invention or a pharmaceutical composition of the present invention.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "higher than 150 mg per day". Furthermore, such an interpretation should apply regardless of the breadth of the range or the characteristics being described.

As used herein and throughout the entire description, the term "alkyl" refers to a monoradical of a saturated straight or branched hydrocarbon. Preferably, the alkyl group comprises from 1 to 12 (such as 1 to 10) carbon atoms, i.e., 1, 2, 3, 4, 5, 6, 7, 8, 9, 10, 11, or 12 carbon atoms (such as 1, 2, 3, 4, 5, 6, 7, 8, 9, or 10 carbon atoms), more preferably 1 to 8 carbon atoms, such as 1 to 6 or 1 to 4 carbon atoms. In some embodiments, the alkyl group employed in the invention contains 1-20 carbon atoms (C₁₋₂₀ alkyl). In another embodiment, the alkyl group employed contains 1-15 carbon atoms (C₁₋₁₅ alkyl). In another embodiment, the alkyl group employed contains 1-10 carbon atoms (C₁₋₂₀ alkyl). In another embodiment, the alkyl group employed contains 1-8 carbon atoms (C₁₋₈ alkyl). In another embodiment, the alkyl group employed contains 1-6 carbon atoms (C₁₋₆ alkyl). In another embodiment, the alkyl group employed contains 1-5 carbon atoms (C₁₋₅ -alkyl). In another embodiment, the alkyl group employed contains 1-4 carbon atoms (C₁₋₄ alkyl). In another embodiment, the alkyl group employed contains 1-3 carbon atoms (C₁₋₃ alkyl). In another embodiment, the alkyl group employed contains 1-2 carbon atoms (C₁₋₂ alkyl). In another embodiment, the alkyl group employed is methyl. Examples of alkyl radicals include, but are not limited to, methyl, ethyl, propyl, iso-propyl, butyl, iso-butyl, tert-butyl, n-pentyl, iso-pentyl, sec-pentyl, neo-pentyl, 1,2-dimethyl-propyl, iso-amyl, n-hexyl, iso-hexyl, sec-hexyl, n-heptyl, iso-heptyl, n-octyl, 2-ethylhexyl, n-nonyl, n-decyl, n-undecyl, n-dodecyl, and the like, which may bear one or more substituents. Alkyl group substituents include, but are not limited to, any of the substituents described herein, that result in the formation of a stable moiety. In some embodiments the alkyl chain is linear. In some embodiments the alkyl chain is branched. In some embodiments the alkyl chain is substituted. In some embodiments the alkyl chain is unsubstituted. In some embodiments the alkyl chain is linear and substituted or unsubstituted. In some embodiments the alkyl chain is branched and substituted or unsubstituted.

As used herein and throughout the entire description, the term "alkoxy" refers to an alkyl group connected to an oxygen atom. Examples are methoxy -OCH₃, or ethoxy -OCH₂CH₃ and also 2-methoxyethoxy.

As used herein and throughout the entire description, the term "haloalkyl" refers to an alkyl group substituted by one halogen substituent up to per halo-substitution. The halogen substituent is preferably fluorine, or chlorine. The haloalkyl is preferably a perfluoroalkyl. In some embodiments, the haloalkyl group employed in the invention contains 1-6 carbon atoms (C₁₋₆ haloalkyl). In another embodiment, the haloalkyl group employed contains 1-5 carbon atoms (C₁₋₅ haloalkyl). In another embodiment, the haloalkyl group employed contains 1-4 carbon atoms (C₁₋₄ haloalkyl). In another embodiment, the haloalkyl group employed contains 1-3 carbon atoms (C₁₋₃ haloalkyl). In another embodiment, the haloalkyl group employed contains 1-2 carbon atoms (C₁₋₂ haloalkyl). In another embodiment, the haloalkyl group employed contains 1-carbon atom (C₁ haloalkyl). In another embodiment, the haloalkyl group employed is trifluoromethyl. Exemplary fluoro-substituted C₁-C₂ alkyl includes -CFH₂, -CF₂H, -CF₃, CH₂CH₂F, -CH₂CHF₂, -CHFCH₃, -CHFCH₃, -CF₂CHF₂. Perfluoro-substituted C₁-C₂ haloalkyl, for example include -CF₃, and -CF₂CF₃. In some embodiments haloalkyl is C₁ haloalky, in particular -CF₃.

As used herein and throughout the entire description, the term "haloalkoxy" refers to a haloalkyl group connected to an oxygen atom. For example, -OCF₃.

As used herein and throughout the entire description, the term "cycloalkyl" represents cyclic non-aromatic versions of "alkyl" with preferably 3 to 14 carbon atoms, such as 3 to 10 carbon atoms, i.e., 3, 4, 5, 6, 7, 8, 9, or 10 carbon atoms, more preferably 3 to 8 carbon atoms, even more preferably 3 to 7 carbon atoms. In certain embodiments, the cycloalkyl group employed in the invention contains 3-14 carbon atoms (C₃₋₁₄ cycloalkyl). In certain embodiments, the cycloalkyl group employed in the invention contains 3-12 carbon atoms (C₃₋₁₂ cycloalkyl). In another embodiment, the cycloalkyl group employed in the invention contains 3-10 carbon atoms (C₃₋₁₀ cycloalkyl). In another embodiment, the cycloalkyl group employed in the invention contains 3-8 carbon atoms (C₃₋₈ cycloalkyl). In another embodiment, the cycloalkyl group employed in the invention contains 3-7 carbon atoms (C₃₋₇ cycloalkyl). In another embodiment, the cycloalkyl group employed in the invention contains 3-6 carbon atoms (C₃₋₆ cycloalkyl). In another embodiment, the cycloalkyl group employed in the invention contains 3-5 carbon atoms (C₃₋₅ cycloalkyl). In another embodiment, the cycloalkyl group employed in the invention contains 3-4 carbon atoms (C₃₋₄ cycloalkyl). In another embodiment, the cycloalkyl group employed in the invention contains 3 carbon atoms (C₃ cycloalkyl). Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include C₃-C₈-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl. Cycloalkyl group substituents include, but are not limited to, any of the substituents described herein, that result in the formation of a stable moiety.

As used herein and throughout the entire description, the term "substituted" indicates that a group, such as an alkyl, a cycloalkyl, a cycloalkene, a heterocyclic moiety, may be substituted with one or more substituents, such as substituted with one or more of C₁-C₁₀ alkyl, C₃-C₇ cycloalkyl, oxo, -OH, -CN, -NO₂, aryl, heteroaryl and heterocyclic. "Substituted" in reference to a group indicates that one or more hydrogen atoms attached to a member atom within the group is replaced with a substituent selected from the group of defined or suitable substituents. It should be understood that the term "substituted" includes the implicit provision that such substitution be in accordance with the permitted valence of the substituted atom and the substituent, and that the substitution results in a stable compound. When it is stated that a group may contain one or more substituents, one or more member atom within the group may be substituted. In addition, a single member atom within the group may be substituted with more than one substituent as long as such substitution is in accordance with the permitted valence of the atom.

### Antibacterial compounds

As outlined above, the present invention provides a compound having a structure according to formula I 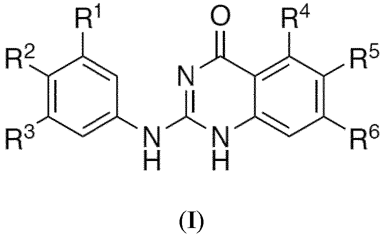 wherein
**R¹**, **R²** and **R³** are each independently selected from H, halogen, cyano, substituted or unsubstituted (C₁-C₆)alkyl, and (C₁-C₆)haloalkyl, under the proviso that not all of **R¹** to **R³** are each H, or
**R² and R³** are taken together to form a substituted or unsubstituted cyclic acetal,
**R⁴**, **R⁵** and **R⁶** are each independently selected from H, -O**R⁷**, (C₁-C₆)alkoxy, halogen, substituted or unsubstituted (C₁-C₆)alkyl, (C₁-C₆)haloalkyl, cycloalkyl, substituted or unsubstituted (C₆-C₁₄)aryl(C₁-C₆)alkyl, and - C(O)-**X-R⁸** under the proviso that not all of **R⁴** to **R⁶** are each H, or
**R⁵ and R⁶** are taken together to form a substituted or unsubstituted cyclic acetal;
**R⁷** is selected from H, (C₁-C₆)alkyl, (C₁-C₆)haloalkyl, and substituted or unsubstituted (C₆-C₁₄)aryl,
**X** is O or NH,
**R⁸** is selected from substituted or unsubstituted (C₁-C₆)alkyl, cycloalkyl, (C₁-C₆)haloalkyl and substituted or unsubstituted (C₆-C₁₄)aryl;
or a pharmaceutically acceptable salt, solvate or hydrate thereof.

In a preferred embodiment, a compound having a structure according to formula I 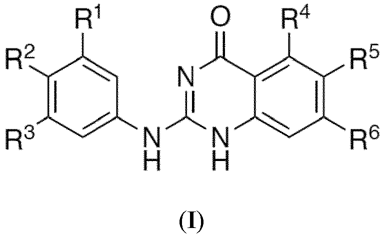 is a compound wherein
**R¹**, **R²** and **R³** are each independently selected from H, halogen, and (C₁-C₆)haloalkyl, under the proviso that not all of **R¹** to **R³** are each H, or
**R² and R³** are taken together to form a substituted or unsubstituted cyclic acetal;
**R⁴**, **R⁵** and **R⁶** are each independently selected from H, -O**R⁷**, (C₁-C₆)alkoxy, halogen, (C₁-C₆)haloalkyl, cycloalkyl, and - C(O)-**X-R⁸** under the proviso that not all of **R⁴** to **R⁶** are each H, or
**R⁵ and R⁶** are taken together to form a substituted or unsubstituted cyclic acetal;
**R⁷** is selected from H, and (C₁-C₆)alkyl,
**X** is O or NH,
**R⁸** is selected from substituted or unsubstituted (C₁-C₆)alkyl, and cycloalkyl;
or a pharmaceutically acceptable salt, solvate or hydrate thereof.

In a preferred embodiment, a compound having a structure according to formula I 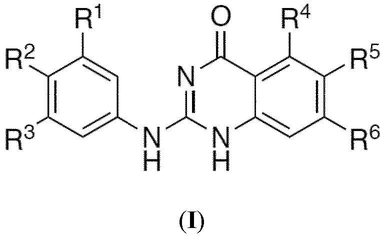 is a compound wherein
**R¹** is H or haloalkyl,
**R²** is H or halogen,
**R³** is H or haloalkyl,
under the proviso that not all of **R¹** to **R³** are each H, or
**R² and R³** are together difluoro acetal,
**R⁴** is selected from H, halogen, haloalkyl, or cycloalkyl,
**R⁵** is selected from H, OH, alkoxy, halogen, cycloalkyl, or - C(O)-**X-R⁸**
   wherein **X** is O or NH, and **R⁸** is selected from alkyl or
   cycloalkyl;
**R⁶** is selected from H, OH, alkoxy, halogen, or haloalkyl, or
**R⁵ and R⁶** are together difluoro acetal;
under the proviso that not all of **R⁴** to **R⁶** are each H,
or a pharmaceutically acceptable salt, solvate or hydrate thereof.

In a preferred embodiment, a compound of formula I has a structure, wherein
**R¹** is H or (C₁-C₆)haloalkyl,
**R²** is H or halogen,
**R³** is H or (C₁-C₆)haloalkyl,
under the proviso that not all of **R¹** to **R³** are each H, or **R² and R³** are together difluoro acetal,
**R⁴** is selected from H, halogen, (C₁-C₆)haloalkyl, or cycloalkyl,
**R⁵** is selected from H, OH, (C₁-C₆)alkoxy, halogen, cycloalkyl, or - C(O)-**X-R⁸**
   wherein **X** is O or NH, and **R⁸** is selected from (C₁-C₆)alkyl or
   cycloalkyl;
**R⁶** is selected from H, OH, (C₁-C₆)alkoxy, halogen, or (C₁-C₆)haloalkyl, or
**R⁵ and R⁶** are together difluoro acetal;
under the proviso that not all of **R⁴** to **R⁶** are each H.

In a preferred embodiment, a compound of formula I has a structure, wherein **R⁴** is C1-haloalkyl, preferably CF₃.

In a preferred embodiment, a compound of formula I has a structure, wherein **R⁴** is H, C1-haloalkyl, preferably CF₃, or cyclohexyl.

In a preferred embodiment, a compound of formula I has a structure, wherein **R²** or **R⁵** is Cl.

In a preferred embodiment, a compound of formula I has a structure, wherein **R⁵** is H, OH, methoxy, or cyclohexyl.

In a preferred embodiment, a compound of formula I has a structure, wherein **R⁶** is H, OH, methoxy, F, C1-haloalkyl, preferably CF₃.

In a preferred embodiment, a compound of formula I has a structure, wherein **R² and R³** or **R⁵ and R⁶** are together difluoro acetal.

In a preferred embodiment, a compound of formula I has a structure, wherein
**R⁵** is - C(O)-**X-R⁸** with **X** being O and **R⁸** being alkyl, preferably butyl;
   or
**R⁵** is - C(O)-**X-R⁸** with **X** being NH and **R⁸** being cycloalkyl, preferably bicyclo[1.1.1]pentyl.

In a preferred embodiment, a compound of formula I has a structure, wherein
**R⁵** and **R⁶** are both OH or both methoxy.

In a preferred embodiment, a compound of formula I has a structure, wherein
**R⁴** and **R⁶** are both halogen, preferably F and **R⁵** is - C(O)-**X-R⁸** with **X** being NH, and **R⁸** being cycloalkyl, preferably bicyclo[1. 1.1]pentyl.

Preferred compounds of formula I are:

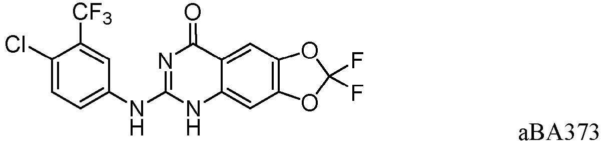

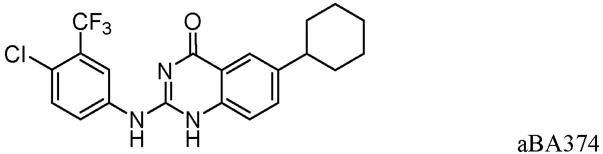

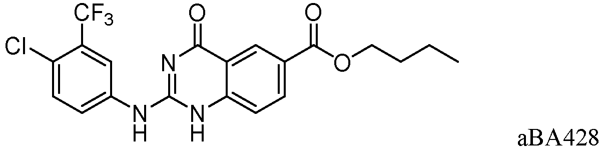

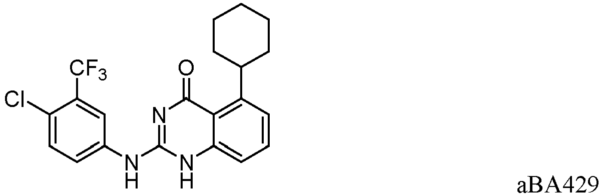

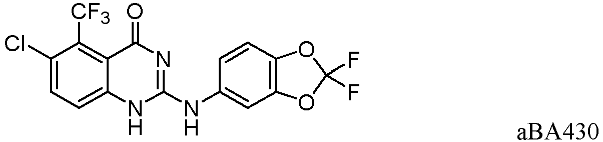

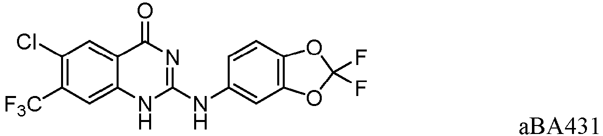

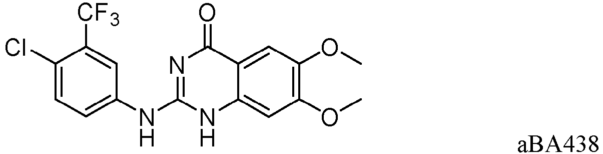

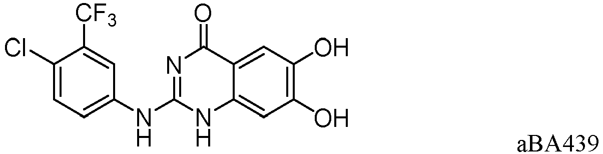

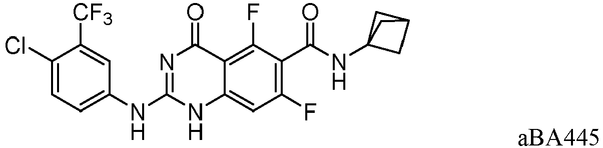

More preferably, a compound of the present invention has a structure selected from

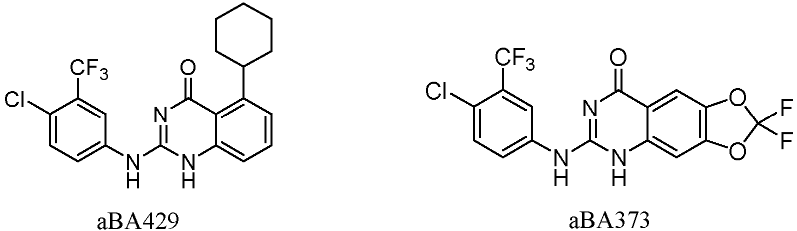

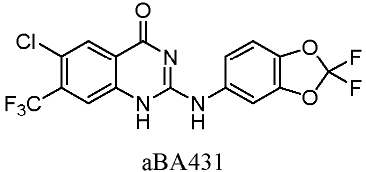

As discussed above, the present invention provides a pharmaceutical composition comprising
(i) at least one compound according to the present invention,
(ii) optionally, pharmaceutically excipient(s) and/or carrier.

Pharmaceutical compositions or preparations may be selected according to the invention from the group of formulations containing tablets, layered tablets, coated tablets, pills, soft or hard capsules, microcapsules, oral retardant drug forms, transdermal systems, suppositories, micro- and nanocrystalline formulations, liposomal formulations, drops, nasal drops, sprays, emulsions, dispersions, solutions, sterile solutions, lyophilisates, powders and inhalation sprays.

The application or use of the pharmaceutical composition or preparation according to the invention is preferably selected from the group comprising oral, peroral, sublingual, buccal, subcutaneous, intravenous, dermal, pulmonary or nasal application or use.

Pharmaceutical compositions or preparations are preferably offered as sterile solutions or lyophilisates, parenteral, peroral and oral retardant drug forms, transdermal systems, micro- and nanocrystalline formulations, liposomal formulations, microcapsules, emulsions, dispersions and are particularly suitable for subcutaneous, intravenous, dermal, transdermal, oral, peroral or pulmonary use or application.

Lactose, starch, sorbitol, mannitol, sucrose, ethyl alcohol and water can be used, for example, as pharmacologically and chemically compatible carriers, solvents or auxiliary agents.

Furthermore, starch, modified starch, gelatine, natural sugars, natural or synthetic polymers such as acacia gum, guar, sodium alginate, carboxymethyl cellulose or polyethylene glycol may be included as binders. Cyclodextrins, modified cyclodextrins, as well as benzoates, chlorides, acetates, tartrates may be contained as stabilizers and stearates, polyethylene glycol, amino acids such as leucine may be used as auxiliaries usually in concentrations of 0.05 % to 15 %. Liquid formulations include solutions, dispersions and emulsions. Liquid formulations for parenteral use are sterile and contain water or water and solubilizers, such as propylene glycol, micelle and mixed micelle formers. Starch or modified starch, alginates, aluminates, bentonites or microcrystalline cellulose can be used as a liquid preparation usually in concentrations between 2% and 30% by weight.

Sugars, sugar alcohols, corn, rice or potato starch, gelatine, gum arabic, tragacanth sugar, ammonium calcium alginate carboxymethyl cellulose, hydroxy propyl methyl cellulose, polyvinyl pyrrolidone as well as inorganic substances can be used as auxiliary agents usually in concentrations between 1% and 30% by weight. Pharmaceutical preparations for subcutaneous, intravenous and transdermal use as well as parenteral and oral modified release dosage forms are claimed as preferred formulations. Such formulations usually consist of a matrix, in particular a matrix with polymers, in many cases biodegradable polymers as a shaping, constituent additive, into which at least one of the compounds of the present invention is incorporated.

The term "pharmaceutically acceptable salts" as used herein includes salts of the compound of the general formula I which are prepared with relatively nontoxic (i.e. pharmaceutically acceptable) acids or bases, depending on the particular substituents found on the compounds of the present invention. If, for example, compounds of the present invention contain acidic functionalities, base addition salts may be obtained by contacting the neutral form of such compounds with a sufficient amount of the desired base, either neat or in a suitable inert solvent. Non-limiting examples of pharmaceutically acceptable base addition salts include sodium, potassium, calcium, ammonium, organic amino, or magnesium salt, or a similar salt. If compounds of the present invention contain basic functionalities, acid addition salts may be obtained by contacting the neutral form of such compounds with a sufficient amount of the desired acid, either neat or in a suitable inert solvent. Non-limiting examples of pharmaceutically acceptable acid addition salts include those derived from inorganic acids like hydrochloric, hydrobromic, nitric, carbonic, phosphoric, partially neutralized phosphoric acids, sulfuric, partially neutralized sulfuric, hydroiodic, or phosphorous acids and the like, as well as the salts derived from relatively nontoxic organic acids like acetic, propionic, isobutyric, maleic. malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the present invention may contain both basic and acidic functionalities that allow the compounds to be converted into either base or acid addition salts. Contacting the salt with a base may regenerate the neutral forms of the compounds of the present invention or acid and isolating the parent compound in the conventional manner. The parent form of the compound differs from the various salt forms in certain physical properties, such as solubility in polar solvents, but otherwise the salts are equivalent to the parent form of the compound for the purposes of the present invention. The compounds of the present invention may possess chiral or asymmetric carbon atoms (optical centers) and/or double bonds. The racemates, diastereomers, geometric isomers and individual optical isomers are encompassed by the present invention. The compounds of the present invention may exist in unsolvated forms as well as solvated forms, including hydrated forms. In general, the solvated forms are equivalent to unsolvated forms and are also encompassed by the present invention. The compounds of the present invention may furthermore exist in multiple crystalline or amorphous forms.

The compounds of the present invention may further be in a so-called prodrug form. Prodrugs of the compounds of the invention are those compounds that readily undergo chemical changes under physiological conditions to provide the compounds of the present invention. Additionally, prodrugs can be converted to the compounds of the present invention by chemical or biochemical methods in an ex-vivo environment. For example, prodrugs can be slowly converted to the compounds of the present invention when, for example, placed in a transdermal patch reservoir with a suitable enzyme or chemical reagent.

### Medical uses of the compounds

As discussed above, the present invention provides the compound of the present invention or the pharmaceutical composition of the present invention for use as a medicament.

As discussed above, the present invention provides the compound of the present invention or the pharmaceutical composition of the present invention for use in the treatment of a bacterial disease or bacterial infection.

Preferably, the bacterial disease or bacterial infection is caused by at least one bacteria selected from the group consisting of *Listeria monocytogenes, Listeria welshimeri, Staphylococcus aureus,* MRSA and clinical isolates thereof; Vancomycin-intermediate *Staphylococcus aureus,* Vancomycin-resistant *Staphylococcus aureus, Staphylococcus haemolyticus, Staphylococcus hominis, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroids, Rhodococcus equi and Burkholderia thailandensis,* preferably wherein the bacterial disease is caused by gram positive bacteria.

In a preferred embodiment, the compound of the present invention or pharmaceutical composition of the present invention is provided for use in the treatment of a bacterial disease or bacterial infection, wherein the bacterial disease or bacterial infection is caused by a methicillin-resistant *Staphylococcus aureus* strain (MRSA).

As discussed above, the present invention provides a disinfectant comprising at least one compound of the present invention.

As discussed above, the present invention provides a method for the treatment of a bacterial disease or bacterial infection.

Said method comprises the step of administering to a subject in need thereof a compound of the present invention or a pharmaceutical composition of the present invention.

Preferably, a therapeutically effective amount of a compound of the present invention or a pharmaceutical composition of the present invention is administered to the subject.

A "therapeutically amount" or "therapeutically effective amount", both of which terms are used herein interchangeably, of a compound of the present invention or a pharmaceutical composition of the present invention is the amount which results in the desired therapeutic result.

The present invention also provides the use of the compound of the present invention or the use of the pharmaceutical composition of the present invention for the manufacture of a medicament.

The present invention also provides the use of the compound of the present invention or the use of the pharmaceutical composition of the present invention for the manufacture of a medicament for the treatment of a bacterial disease or bacterial infection.

Preferably, the bacterial disease or bacterial infection is caused by at least one bacteria selected from the group consisting of *Listeria monocytogenes, Listeria welshimeri, Staphylococcus aureus,* MRSA and clinical isolates thereof; Vancomycin-intermediate *Staphylococcus aureus,* Vancomycin-resistant *Staphylococcus aureus, Staphylococcus haemolyticus, Staphylococcus hominis, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroids, Rhodococcus equi and Burkholderia thailandensis,* preferably wherein the bacterial disease is caused by gram positive bacteria.

In a preferred embodiment, the compound of the present invention or pharmaceutical composition of the present invention is used for the manufacture of a medicament for the treatment of a bacterial disease or bacterial infection, wherein the bacterial disease or bacterial infection is caused by a methicillin-resistant *Staphylococcus aureus* strain (MRSA).

The following examples and drawings illustrate the present invention without, however, limiting the same thereto.

### BRIEF DESCRIPTION OF THE DRAWINGS

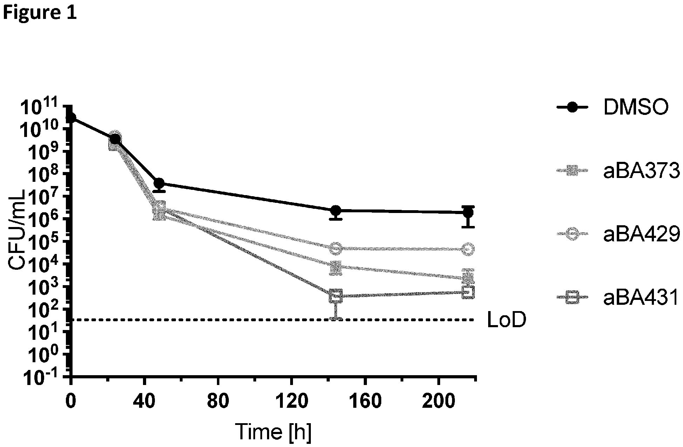

**Figure 1** shows a persister cell assay.
   A population of bacterial persisters of *S. aureus* ATCC29213 is eradicated after 5 to 7 days of treatment with 8x MIC of tested compounds. Persisters are generated from a stationary culture by treatment with 100x MIC of ciprofloxacin for 24 h. Ciprofloxacin is removed by washing and the bacteria are transferred to PBS + 1% (v/v) MH2-medium and treated with the test compound. Rifampicin was used at 10x MIC as a control. LoD = limit of detection.

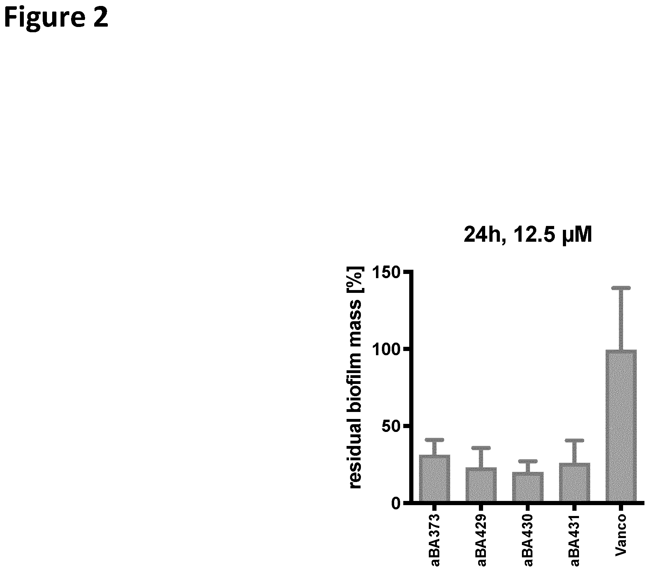

**Figure 2** shows the results of a biofilm eradication assay.
   Biofilms of *S. aureus* ATCC29123 are grown in 96-well plates and treated with compound for 24h. Afterwards compound is removed by washing and the residual biofilm is stained with crystal violet. The treated biofilms are normalized to a DMSO-treated vehicle control. Vancomycin was used as a reference antibiotic. All tested compounds eradicated more than 50% of the bacterial biofilms at a concentration of 12.5 µM.

### EXAMPLES

### Example 1 Synthesis

### Quinazolinones were obtained either

by reaction of an *N-*arylcyanamide with an 2-aminobenzoic acid methyl ester derivative (aBA373): 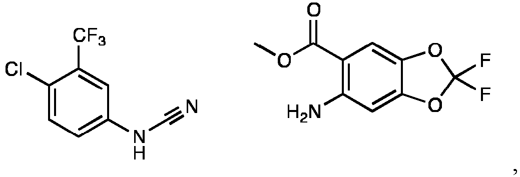 or by using a 2-chloroquinazolin-4-ol derivative together with the corresponding aniline (aBA430, aBA431), such as:

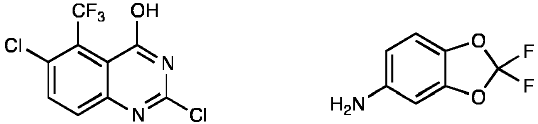

The compounds can be synthesized with the general knowledge of an organic chemist as outlined in, for example, Smith, Michael B. March's Advanced Organic Chemistry (2019) and Schwetlick, Klaus: Organikum (2015).

### Example 2 Biological testing

The compounds of the present invention were tested against numerous bacterial strains. Tables 1 and 2 below summarize the results and indicates the minimal inhibitory concentration (MIC) values of the compounds against the indicated bacterial strains.

As can be taken from Tables 1 and 2, the compounds show excellent activity against a broad range of gram-positive bacteria with MIC values mostly in the sub-micromolar range.

Activity against mycobacteria is a promising starting point for possible future applications for the treatment of tuberculosis. Intriguingly, the compounds also show high antibacterial activity against gram-negative *E. coli* RFM795, while they are inactive against all other gram-negative strains tested. *E. coli* RFM795 are highly deficient in their lipopolysaccharide biosynthesis, which makes them permeable for small molecules. This indicates that the compounds are potentially effective against many gram-negative bacteria but fail to reach their target in wild-type strains due to a lack of permeability. Of note, some compounds show moderate activity in *A. baumannii,* demonstrating that it is possible to make this compound class more permeable towards gram-negative bacteria by according structural modifications.

It is to be noted that the compounds are also active against persister cells and biofilms, two bacterial populations that are very hard to treat in the clinic (Figures 1 and 2). Development of a systemic antibiotic active against such bacterial populations is still an unmet medical need.

**Table 1**

| **Compound ID** | **MIC *S. aureus* ATCC 29213 [µM]** | **MIC *S. aureus* DSM18827 [µM]** | **MIC *S. aureus* Mu50 [µM]** | **MIC *S. aureus* USA300 [µM]** |
|---|---|---|---|---|
| **aBA373** | 1.56 | 1.56 | 1.56 | 1.56 |
| **aBA429** | 0.78 | 0.78 | 0.39-0.78 | 0.78 |
| **aBA430** | 1.56 | 1.56 | 1.56 | 1.56 |
| **aBA431** | 1.56 | 1.56-3.13 | 1.56-3.13 | 1.56 |

**Table 2**

| **Compound ID** | **MIC *E. faecium* DSM20477 [µM]** | **MIC *E. faecium* DSM17050 [µM]** | **MIC *E. faecalis* V583 [µM]** | **MIC *A. baumanii* DSM30007 [µM]** | **MIC *E. coli* MM28 [µM]** | **MIC *E. coli* RFM795 [µM]** |
|---|---|---|---|---|---|---|
| **aBA373** | 3.13-6.25 | 3.13 | 3.13 | >50 | >25 | 3.13-6.25 |
| **aBA429** | 1.56 | 3.13 | 1.56 | >100 | >25 | >25 |
| **aBA430** | >25 | >25 | >25 | >25 | >25 | 3.13 |
| **aBA431** | 3.13 | 3.13 | 3.13 | >100 | >25 | 3.13 |

The features disclosed in the foregoing description, in the claims and/or in the accompanying drawings may, both separately and in any combination thereof, be material for realizing the invention in diverse forms thereof.

### REFERENCES

Beaver et al., J. Am. Chem. Soc. 79, 1236-1245, 1957.
Cassini, A. et al. Lancet Infect. Dis. 3099, 1-11, 2018.
Chang, H. C. et al., J. Antimicrob. Chemother. 71, 449-459, 2016.
European Commission. SCIENTIFIC COMMITTEE ON CONSUMER PRODUCTS SCCP Opinion on Triclocarban, 2005.
Lakemeyer et al., Angew. Chemie Int. Ed. 14440-14475, 2018.
Proctor, R. A. et al., Antimicrob. Agents Chemother. 2, 801-803, 2002.
Pujol, E. et al. Molecules 23, 2853, 2018.
Schwetlick, Klaus: Organikum. 24th Edition. Wiley-VCH, ISBN: 978-3-527-33968-6 April 2015, 914 pages.
Seth, P. P. et al. Bioorganic Med. Chem. Lett. 14, 5569-5572, 2004.
Smith, Michael B. March's Advanced Organic Chemistry: Reactions, Mechanisms, and Structure, 8th Edition. ISBN: 978-1-119-37179-3 November 2019, 2144 pages.
Yoon, J. et al. KR 20030080509 A; Antibacterial Compositions Containing Urea, Thiourea and Sulfamide Derivatives. 1-14, 2002.

## Claims

1. A compound having a structure according to formula I 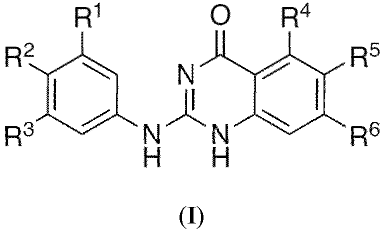 wherein
**R¹**, **R²** and **R³** are each independently selected from H, halogen, cyano, substituted or unsubstituted (C₁-C₆)alkyl, and (C₁-C₆)haloalkyl, under the proviso that not all of **R¹** to **R³** are each H, or
**R²** and **R³** are taken together to form a substituted or unsubstituted cyclic acetal;
**R⁴**, **R⁵** and **R⁶** are each independently selected from H, -O**R⁷**, (C₁-C₆)alkoxy, halogen, substituted or unsubstituted (C₁-C₆)alkyl, (C₁-C₆)haloalkyl, cycloalkyl, substituted or unsubstituted (C₆-C₁₄)aryl(C₁-C₆)alkyl, and - C(O)-**X-R⁸** under the proviso that not all of **R⁴** to **R⁶** are each H, or
**R⁵ and R⁶** are taken together to form a substituted or unsubstituted cyclic acetal;
**R⁷** is selected from H, (C₁-C₆)alkyl, (C₁-C₆)haloalkyl, and substituted or unsubstituted (C₆-C₁₄)aryl,
**X** is O or NH,
**R⁸** is selected from substituted or unsubstituted (C₁-C₆)alkyl, cycloalkyl, (C₁-C₆)haloalkyl and substituted or unsubstituted (C₆-C₁₄)aryl;
or a pharmaceutically acceptable salt, solvate or hydrate thereof.

2. The compound of claim 1, wherein
**R¹**, **R²** and **R³** are each independently selected from H, halogen, and (C₁-C₆)haloalkyl, under the proviso that not all of **R¹** to **R³** are each H, or
**R² and R³** are taken together to form a substituted or unsubstituted cyclic acetal;
**R⁴**, **R⁵** and **R⁶** are each independently selected from H, -O**R⁷**, (C₁-C₆)alkoxy, halogen, (C₁-C₆)haloalkyl, cycloalkyl, and - C(O)-**X-R⁸** under the proviso that not all of **R⁴** to **R⁶** are each H, or
**R⁵ and R⁶** are taken together to form a substituted or unsubstituted cyclic acetal;
**R⁷** is selected from H, and (C₁-C₆)alkyl,
**X** is O or NH,
**R⁸** is selected from substituted or unsubstituted (C₁-C₆)alkyl, and cycloalkyl.

3. The compound of claim 1 or 2, wherein
**R¹** is H or (C₁-C₆)haloalkyl,
**R²** is H or halogen,
**R³** is H or (C₁-C₆)haloalkyl,
under the proviso that not all of **R¹** to **R³** are each H,
or
**R² and R³** are together difluoro acetal,
**R⁴** is selected from H, halogen, (C₁-C₆)haloalkyl, or cycloalkyl,
**R⁵** is selected from H, OH, (C₁-C₆)alkoxy, halogen, cycloalkyl, or - C(O)-**X-R⁸**
wherein **X** is O or NH, and **R⁸** is selected from (C₁-C₆)alkyl or
cycloalkyl;
**R⁶** is selected from H, OH, (C₁-C₆)alkoxy, halogen, or (C₁-C₆)haloalkyl, or
**R⁵ and R⁶** are together difluoro acetal;
under the proviso that not all of **R⁴** to **R⁶** are each H.

4. The compound of any one of claims 1 to 3, wherein
**R¹** is C1-haloalkyl, preferably CF₃,
and/or
**R⁴** is H, F, C1-haloalkyl, preferably CF₃, or cyclohexyl,
and/or
**R²** or **R⁵** is Cl,
and/or
**R⁵** is H, OH, methoxy, or cyclohexyl,
and/or
**R⁶** is H, OH, methoxy, F, C1-haloalkyl, preferably CF₃,
and/or
**R² and R³** or **R⁵ and R⁶** are together difluoro acetal.

5. The compound of any one of claims 1 to 4, wherein
**R⁵** is - C(O)-**X-R⁸** with **X** being O and **R⁸** being alkyl, preferably butyl;
or
**R⁵** is - C(O)-**X-R⁸** with **X** being NH and **R⁸** being cycloalkyl, preferably bicyclo[1.1.1]pentyl.

6. The compound of any one of claims 1 to 5, wherein
**R⁵** and **R⁶** are both OH or both methoxy;
or
**R⁴** and **R⁶** are both halogen, preferably F and **R⁵** is - C(O)-**X-R⁸** with **X** being NH, and **R⁸** being cycloalkyl, preferably bicyclo[1.1.1]pentyl.

7. The compound of any one of claims 1 to 6 having a structure selected from

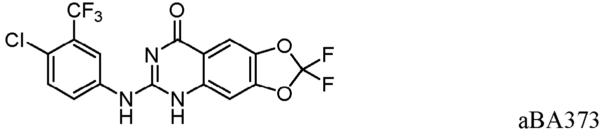

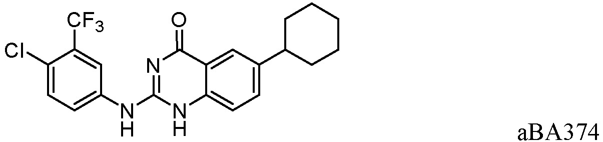

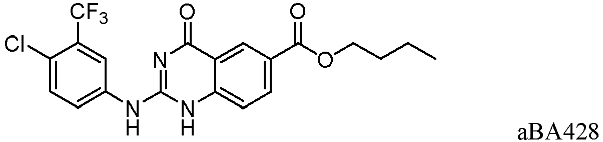

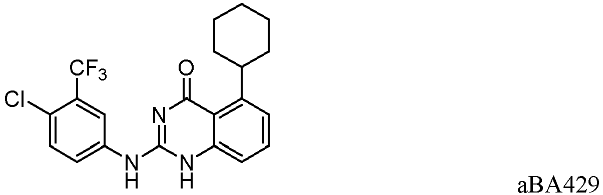

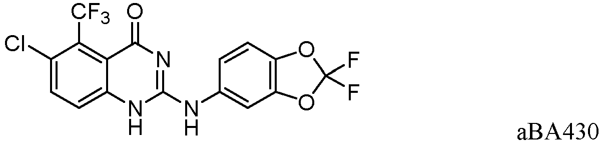

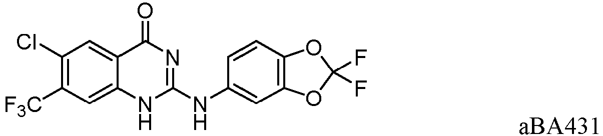

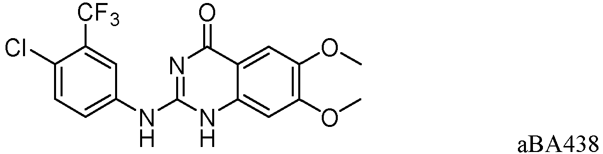

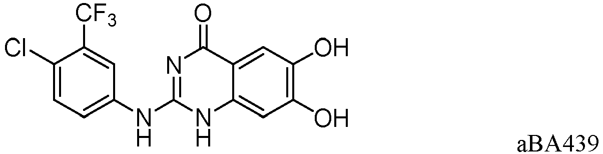

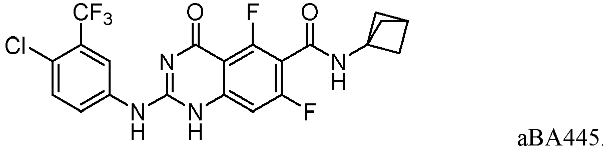

8. The compound of any one of claims 1 to 7 having a structure selected from

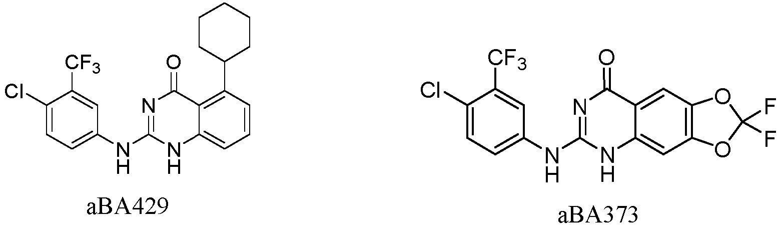

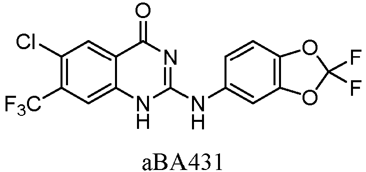

9. A pharmaceutical composition comprising
(i) at least one compound according to any one of claims 1 to 8,
(ii) optionally, pharmaceutically excipient(s) and/or carrier.

10. The compound of any one of claims 1 to 8 or the pharmaceutical composition of claim 8 for use as a medicament.

11. The compound of any one of claims 1 to 8 or the pharmaceutical composition of claim 9 for use in the treatment of a bacterial disease or bacterial infection.

12. The compound or pharmaceutical composition for use according to claim 11, wherein the bacterial disease or bacterial infection is caused by at least one bacteria selected from the group consisting of *Listeria monocytogenes, Listeria welshimeri, Staphylococcus aureus,* MRSA and clinical isolates thereof; Vancomycin-intermediate *Staphylococcus aureus,* Vancomycin-resistant *Staphylococcus aureus, Staphylococcus haemolyticus, Staphylococcus hominis, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroids, Rhodococcus equi and Burkholderia thailandensis,* preferably wherein the bacterial disease is caused by gram positive bacteria.

13. The compound or pharmaceutical composition for use according to claim 11 or 12, wherein the bacterial disease or bacterial infection is caused by a methicillin-resistant *Staphylococcus aureus* strain (MRSA).

14. A disinfectant comprising at least one compound of any one of claims 1 to 8.

15. A method for the treatment of a bacterial disease or bacterial infection, comprising the step of administering to a subject in need thereof a compound of any one of claims 1 to 8 or a pharmaceutical composition of claim 9.
